# FASCICULE DE BREVET EUROPEEN

(11) **EP 1 776 983 B1**
(45) Date de publication et mention de la délivrance du brevet: **05.11.2008**
(21) Numéro de dépôt: 06121664.4
(22) Date de dépôt: 03.10.2006
(51) Int. Cl.: A61Q 5/02, A61Q 5/12, A61K 8/73

(54) **Composition de lavage et de conditionnement des matières kératiniques comprenant un carboxyalkylamidon, utilisation et procédé.**
Keratinfasern Reinigungs- und Pflegemittel enthaltend eine Carboxyalkylstärke, Verwendung und Verfahren
Composition for cleansing and conditioning keratinous fibres containing a carboxyalkyl starch, use thereof and process

(30) Priorité: 20.10.2005 FR 0553189
(43) Date de publication de la demande: 25.04.2007
(73) Titulaire: L'Oréal, 75008 Paris (FR)
(72) Inventeur: Parris, Eric, 92110 Clichy (FR); Fack, Géraldine, 92300 Levallois (FR)
(74) Mandataire: Le Blainvaux Bellegarde, Françoise

(56) Documents cités:
- EP-A- 1 462 088
- FR-A- 2 779 648
- US-A- 3 629 121

## Description

La présente invention concerne une composition de lavage et de conditionnement des matières kératiniques, en particulier des fibres kératiniques, comprenant une base tensioactive lavante et plus de 5% d'un carboxyalkylamidon, l'utilisation de ladite composition pour le lavage et le conditionnement des matières kératiniques, en particulier des fibres kératiniques, ainsi qu'un procédé de mise en oeuvre de ladite composition.

Dans le domaine des shampoings conditionneurs, on associe généralement une base lavante et un agent de conditionnement qui peut être un polymère cationique, un polymère amphotère, une silicone, une huile synthétique ou naturelle, un corps gras ou un de leurs mélanges. Ces agents de conditionnement sont utilisés pour améliorer le démêlage et la douceur des cheveux mouillés et séchés mais possèdent un effet limité en ce qui concerne les propriétés de maintien de la chevelure et peuvent avoir tendance à alourdir la chevelure et à la ternir.

Généralement, les compositions lavantes sont épaissies. On recherche des produits que l'on peut doser et prendre aisément dans la main. Pour ce faire, ces produits doivent présenter une certaine consistance ou viscosité. En effet, un produit liquide est beaucoup plus difficile à doser et s'écoule facilement entre les doigts. Par ailleurs, on recherche de nouvelles textures pour les compositions de shampooing.

Pour épaissir les compositions lavantes, on a déjà utilisé des polymères naturels tels que les celluloses. Malheureusement, ces épaississants donnent des compositions instables et/ou qui ne sont pas lisses et homogènes. De plus, ces épaississants présentent l'inconvénient de diminuer la qualité de la mousse et les performances cosmétiques des shampooings, notamment en rendant les cheveux plus chargés et plus rêches. La mousse des compositions épaissies n'est généralement pas suffisamment douce, elle ne se développe pas facilement que ce soit en vitesse ou en abondance.

Ainsi, il subsiste le besoin d'un système épaississant permettant d'épaissir fortement les compositions détergentes sans influer sur les propriétés cosmétiques et moussantes desdites compositions ou en les améliorant.
Les carboxyméthylamidons ont déjà été utilisés comme adjuvant dans les lessives (US3629121).
L'utilisation de carboxyméthylamidon, est également connue en cosmétique. En effet, la demande EP 1188432 décrit l'utilisation de ces amidon comme agents absorbant dans des compositions solides cosmétiques.
Le document EP1588696 décrit des compositions cosmétiques comprenant un tensioactif anionique, jusqu'à 5% en poids de carboxyméthylamidon et de l'eau.
La demanderesse a maintenant découvert que l'utilisation de carboxyalkylamidon dans des compositions lavantes permettait de surmonter les inconvénients indiqués ci-dessus, et d'obtenir ainsi une composition stable d'aspect homogène et esthétique et présentant une texture très épaisse et translucide. La qualité de la mousse est très bonne. Lors de l'application de la composition sur les cheveux mouillés, la mousse démarre facilement et la mousse est abondante et aérée. Ces propriétés permettent d'utiliser une quantité inférieure de shampooing pour un volume de mousse équivalent. Par ailleurs, les propriétés cosmétiques et en particulier le lissage, le gonflant et la souplesse (malléabilité) sont améliorées.

L'invention a donc pour objet une composition de lavage et de conditionnement des matières kératiniques, en particulier des fibres kératiniques, comprenant dans un milieu cosmétiquement acceptable :
- de 3 à 50% en poids par rapport au poids total de la composition d'au moins un ou plusieurs tensioactifs choisis parmi les tensioactifs anioniques, les tensioactifs non ioniques et leurs mélanges,
- plus de 5% en poids par rapport au poids total de la composition d'au moins un carboxyalkylamidon ou l'un de ses sels,
- de 50 à 92% en poids d'eau par rapport au poids total de la composition,
le pH de la composition allant de 3 à 8.

Un autre objet de l'invention est l'utilisation de ladite composition pour le lavage et le conditionnement des matières kératiniques, en particulier des fibres kératiniques, et plus particulièrement des cheveux.

L'invention a encore pour objet un procédé de lavage et de conditionnement des fibres kératiniques mettant en oeuvre la composition selon l'invention.

D'autres objets, caractéristiques, aspects et avantages de l'invention apparaîtront encore plus clairement à la lecture de la description et des divers exemples qui suivent.

Les compositions selon l'invention sont de préférence épaisses, c'est à dire ayant une viscosité allant de 10 Pa.s à 5000 Pa.s, de préférence de 10 à 1000 Pa.s, plus particulièrement de 30 à 500 Pa.s. La viscosité est mesurée à 25°C avec un viscosimètre HAAKE RS1 de THERMO ELECTRON à un taux de cisaillement de 1 s⁻¹. Ce viscosimètre est un viscosimètre à contrainte imposée en géométrie cône plan (par exemple de diamètre 60 mm)

Les carboxyalkylamidons sont de préférence des carboxyalkyl (C1-C4) amidon et plus particulièrement des carboxyméthylamidons.

Les sels sont notamment des sels de métal alcalin ou alcalinoterreux tels que Na, K 1/2, Li, NH₄, d'un ammonium quaternaire ou d'une amine organique telle que la mono, di ou triéthanolamine.

Les amidons sont généralement issus de sources végétales telle que les céréales, les tubercules, les racines, les légumes, les fruits et notamment issus de plantes telles que le blé, le mais, le riz, les pommes de terre, le manioc (tapioca), la banane, les patates douces. L'amidon est de préférence issu de la pomme de terre.

Les amidons sont des polysaccharides comprenant une répétition d'unités anhydroglucose.

Les carboxyalkylamidons sont obtenus par greffage de groupements carboxyalkyl sur une ou plusieurs fonctions alcools de l'amidon, notamment par réaction d'amidon et de monochloroacétate de sodium en milieu alcalin.

Les groupements carboxyalkyl sont généralement fixés par l'intermédiaire d'une fonction éther, plus particulièrement sur le carbone 1.

Le degré de substitution va de préférence de 0,1 à 1 et plus particulièrement de 0,15 à 0,5. Le degré de substitution est défini selon la présente invention comme étant le nombre moyen de groupements hydroxyles substitués par un groupement ester ou éther (en l'occurrence éther pour les carboxyméthylamidons) par unité monosaccharidique du polysaccharide.
Les amidons se présentent notamment sous forme de poudre. Généralement la totalité des particules à un diamètre inférieure à 500 microns.

Les carboxyalkylamidons comprennent de préférence des motifs de formule suivante:

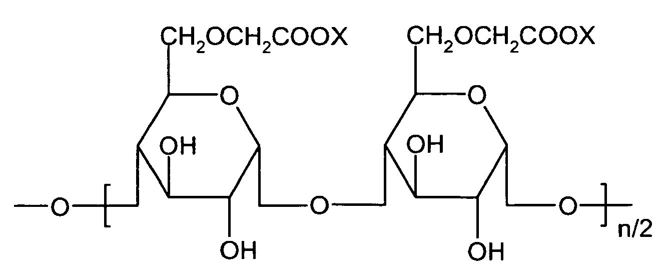

X désigne un atome d'hydrogène, un métal alcalin ou alcalinoterreux tels que Na, K 1/2, Li, NH₄, un ammonium quaternaire ou une amine organique. De préférence X désigne un ion Na+.

Les carboxyalkylamidons utilisables selon la présente invention sont de préférence les carboxyalkylamidons non prégélatinisés.

Les carboxyalkylamidons utilisables selon la présente invention sont de préférence les carboxyalkylamidons réticulés partiellement ou totalement.

Les carboxyalkylamidons utilisables selon la présente invention sont de préférence des sels de sodium de carboxyalkylamidons, en particulier un sel de sodium de carboxyméthylamidon de pomme de terre vendus notamment sous la dénomination PRIMOJEL par la société DMV International. Plus de 95% des particules de cet amidon ont un diamètre inférieur à 100 microns et plus particulièrement inférieur à 65 microns.

Le ou les carboxyalkylamidons de l'invention sont généralement présents en une quantité allant de plus de 5 à 20 % en poids, préférentiellement de plus de 5 à 15% en poids et, mieux encore, de 6 à 12% en poids par rapport au poids total de la composition.

Les tensioactifs anioniques pouvant être utilisés dans la composition sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates polyoxyalkylénés, les alkylamidoéthersulfates polyoxyalkylénés, les alkylaryl-polyéthersulfates polyoxyalkylénés, les monoglycéride-sulfates ; les alkylsulfonates, les alkylamidesulfonates, les alkylarylsulfonates, les α-oléfine-sulfonates, les paraffine-sulfonates, les alkylsulfosuccinates, les alkyléthersulfosuccinates polyoxyalkylénés, les alkylamide-sulfosuccinates, les alkylsulfo-acétates, les acylsarcosinates et les acylglutamates, les groupes alkyle et acyle de tous ces composés comportant de 6 à 24 atomes de carbone, de préférence de 8 à 18 et le groupe aryle désignant de préférence un groupe phényle ou benzyle.
Les tensioactifs polyoxyalkylénés comportent généralement de 1 à 10 motifs oxyde d'éthylène et plus particulièrement de 2 à 5.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates polyoxyalkylénés comportant de 2 à 5 motifs oxyde d'éthylène et les alkyléthercarboxylates, et leurs mélanges, en particulier sous forme de sels de métaux alcalins ou alcalino-terreux, d'ammonium, d'amine ou d'aminoalcool.

Le ou les tensioactifs anioniques sont de préférence présents en une quantité totale allant de 3 à 50 % en poids, mieux encore de 4 à 25% en poids par rapport au poids total de la composition et plus particulièrement de 8 à 20% en poids.

Les tensioactifs non-ioniques utilisables dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitane éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Parmi les tensioactifs non-ioniques cités ci-dessus, on utilise de préférence les (alkyl en C₆₋₂₄)polyglycosides, et plus particulièrement les (alkyl en C₈₋₁₈)polyglycosides.

La composition peut également comprendre en outre au moins un tensioactif amphotère.

Les agents tensioactifs amphotères, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₂-₈)bétaïnes ou les (alkyl en C₈₋₂₀)amido(alkyl en C₂₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (1) et (2):

Rₐ-CONHCH₂CH₂-N(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle;
et

Rₐ'-CONHCH₂CH₂-N(B)(C) (2)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle saturé ou insaturé en C7-C23, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauro-amphodiacétate de disodium, caprylamphodiacétate de disodium, caprylo-amphodiacétate de disodium, cocoamphodipropionate de disodium, lauro-amphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères cités ci-dessus, on utilise de préférence les (alkyl en C₈₋₂₀)-bétaïnes, les (alkyl en C₈₋₂₀)-amido(alkyl en C₂₋₈)bétaïnes, les alkylamphodiacétates et leurs mélanges.

Les tensioactifs non ioniques et/ou amphotères sont de préférence présents dans la composition selon l'invention en une quantité allant de 0,01 à 20 % en poids, mieux encore de 0,5 à 10% en poids par rapport au poids total de la composition et plus particulièrement de 1 à 5% en poids.

La quantité totale de base lavante, à savoir la quantité totale de tensioactifs, va de préférence comprise de 4 à 50 % en poids, mieux encore de 5 à 30 % en poids par rapport au poids total de la composition.

Selon l'invention, le rapport en poids du carboxyalkylamidon à la quantité totale de tensioactifs va de préférence de 0,01 à 2 et de préférence de 0,05 à 1,5 et plus particulièrement de 0,1 à 1,5.

La composition selon l'invention peut notamment comprendre en outre un ou plusieurs polymères cationiques. Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère comprenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 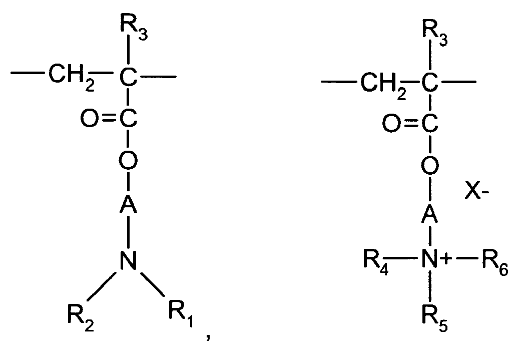 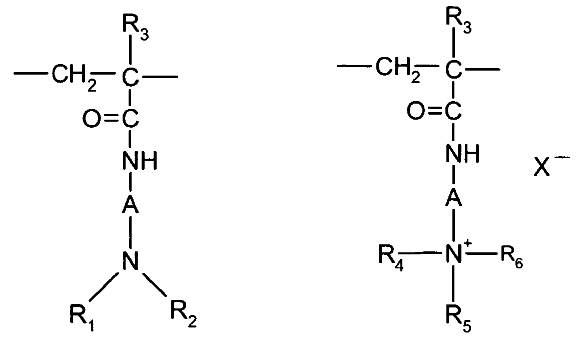 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer:
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/vinylcaprolactame/ vinylpyrrolidone tel que le produit vendu sous la dénomination GAFFIX VC 713 par la société ISP,
   - les copolymères vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthyl-aminopropyle quaternisé tel que le produit vendu sous la dénomination "GAFQUAT HS 100" par la société ISP.
(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
   Les gommes de galactomannane cationiques sont décrites plus particulièrement dans les brevets US 3 589 578 et 4 031 307 en particulier les gommes de guar comprenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société RHODIA.
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (II) ou (III) : 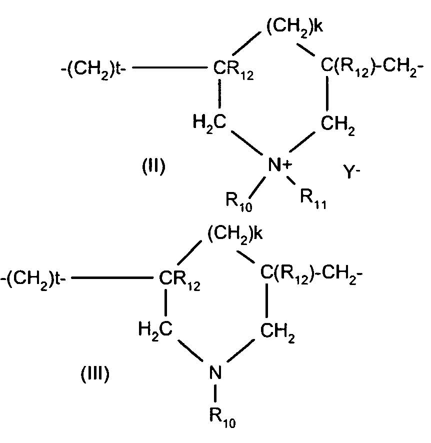 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de sels (par exemple chlorure) de diméthyldiallylammonium vendu sous la dénomination "MERQUAT 100" par la société NALCO (et leurs homologues de faibles masses molaires moyenne en poids) et les copolymères de sels (par exemple chlorure) de diallyldiméthylammonium et d'acrylamide par exemple commercialisés sous la dénomination "MERQUAT 550".
(8) les polymères de diammonium quaternaire comprenant des motifs récurrents répondant à la formule : 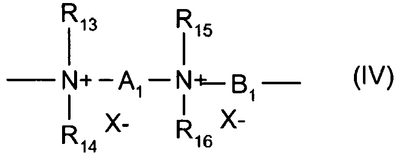 formule (IV) dans laquelle :
   R₁₃, R₁₄, R₁₅ et R₁₆, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles comprenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou-CO-NH-R₁₇-D où R₁₇ est un alkylène et D un groupement ammonium quaternaire ;
   A1 et B1 représentent des groupements polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   n désigne un entier allant de 1 à 8, de préférence 2 à 4
      dans lequel D désigne :
      a) un reste de glycol de formule : -O-Z-O-, où Z désigne un radical hydrocarboné linéaire ou ramifié ou un groupement répondant à l'une des formules suivantes :

         -(CH2-CH2-O)x-CH2-CH2-

         -[CH2-CH(CH3)-O]y-CH2-CH(CH3)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH2-CH2-S-S-CH2-CH2- ;
      d) un groupement uréylène de formule : -NH-CO-NH- ;

   De préférence, X⁻ est un anion tel que le chlorure ou le bromure.
   Ces polymères ont une masse molaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 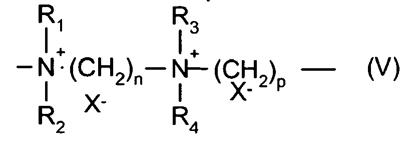 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
   Un composé de formule (V) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(9) les polymères de polyammonium quaternaires comprenant des motifs de formule (VI): 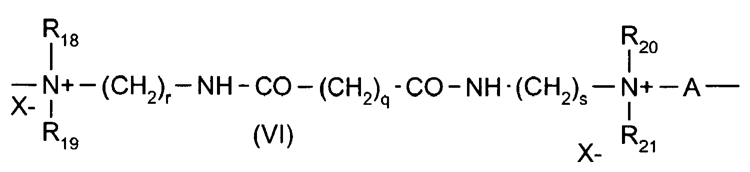 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X- désigne un anion tel qu'un halogènure,
   A désigne un radical d'un dihalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par COGNIS, référencé sous le nom de « POLYETHYLENEGLYCOL (15) TALLOW POLYAMINE » dans le dictionnaire CTFA.
(12) Les polymères de préférence réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50 % en poids dudit copolymère dans de l'huile minérale. Cette dispersion est commercialisée sous le nom de « SALCARE^{®} SC 92 » par la Société CIBA. On peut également utiliser un homopolymère réticulé du chlorure de méthacryloyloxyéthyl triméthylammonium comprenant environ 50 % en poids de l'homopolymère dans de l'huile minérale ou dans un ester liquide. Ces dispersions sont commercialisées sous les noms de « SALCARE^{®} SC 95 » et « SALCARE^{®} SC 96 » par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on peut utiliser de préférence les dérivés d'éther de cellulose quaternaires tels que les produits vendus sous la dénomination « JR 400 » par la Société AMERCHOL, les cyclopolymères cationiques, en particulier les homopolymères ou copolymères de sels (par exemple chlorure) de diméthyldiallylammonium, vendus sous les dénominations « MERQUAT 100 », « MERQUAT 550 » et « MERQUAT S » par la société NALCO et leurs homologues de faibles poids moléculaires en poids, les polymères quaternaires de vinylpyrrolidone et de vinylimidazole, les homopolymères ou copolymères éventuellement réticulés de sels de méthacryloyloxyalkyl(C₁-C₄) trialkyl(C₁-C₄)ammonium et leurs mélanges.

Le ou les polymères cationiques sont généralement présents à des concentrations allant de 0,01 à 20%, de préférence de 0,05 à 10% et plus particulièrement de 0,1 à 5 % en poids par rapport au poids total de la composition.

La composition selon l'invention peut notamment comprendre en outre au moins une silicone.

Les silicones utilisables conformément à l'invention sont en particulier des polyorganosiloxanes insolubles dans la composition et peuvent se présenter sous forme d'huiles, de cires, de résines ou de gommes.

La viscosité des silicones est par exemple mesurée à 25°C selon la norme ASTM 445 Appendice C.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition allant de 60° C à 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique : 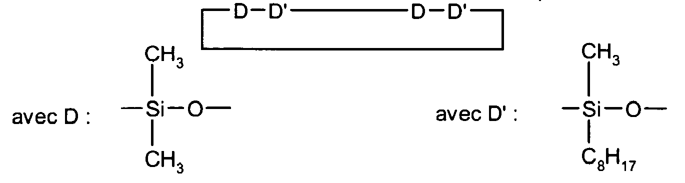 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s.

Parmi ces polyalkylsiloxanes, on peut citer à titre non limitatif les produits commerciaux suivants :
- les huiles de la série MIRASIL commercialisées par la société RHODIA CHIMIE, telles que par exemple l'huile MIRASIL DM 500 000 ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles MIRASIL DPDM de RHODIA CHIMIE ;
- les huiles des séries RHODORSIL 70 633 et 763 de RHODIA CHIMIE ;
- l'huile DOW CORNING 556 COSMETIC GRAD FLUID de DOW CORNING;
- les silicones de la série PK de BAYER comme le produit PK20;
- les silicones des séries PN, PH de BAYER comme les produits PN1000 et PH1000;
- certaines huiles des séries SF de GENERAL ELECTRIC telles que SF 1023,
   SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334.
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.

Selon l'invention, toutes les silicones peuvent également être utilisées sous forme d'émulsions, de microémulsions, de dispersions.

Les silicones particulièrement préférées conformément à l'invention sont :
- les silicones non volatiles choisies dans la famille des polydiméthylsiloxanes à groupements terminaux triméthylsilyle telles que les huiles ayant une viscosité comprise allant de 0,2 à 2,5 m²/s à 25° C telles que les huiles de la séries DC200 de DOW CORNING en particulier celle de viscosité 60 000 cSt, des séries MIRASIL DM et plus particulièrement l'huile MIRASIL DM 500 000 commercialisées par la société RHODIA CHIMIE ou l'huile de silicone AK 300.000 de la société WACKER, les polyalkylsiloxanes à groupements terminaux diméthylsilanol tels que les diméthiconol ou les polyalkylarylsiloxanes tels que l'huile MIRASIL DPDM commercialisée par la société RHODIA CHIMIE ;
- les polydiméthylsiloxanes à groupements aminés notamment les amodiméthicones ou les triméthylsilylamodiméthicones ;

La ou les silicones sont généralement présente à des concentrations allant de 0,01 à 20% en poids, de préférence de 0,05 à 10% en poids, plus particulièrement de 0,1 à 5% et encore plus préférentiellement de 0,5 à 3 % en poids par rapport au poids total de la composition.

Par milieu cosmétiquement acceptable, on entend un milieu compatible avec les matières kératiniques, notamment les fibres kératiniques telles que les cheveux, mais aussi d'odeur, d'aspect et de toucher agréables. Le milieu est de préférence aqueux.

Le milieu aqueux cosmétiquement acceptable est constitué d'eau ou d'un mélange d'eau et d'au moins un solvant organique choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols.

De préférence, la composition comprend de 50 à 90 % en poids d'eau par rapport au poids total de la composition et plus particulièrement de 60 à 85% en poids et encore plus préférentiellement de 65 à 80% en poids.

Le pH des compositions selon l'invention va de préférence de 4 à 8 et, mieux encore de 6 à 8.

La composition selon l'invention peut comprendre en outre des additifs choisis parmi les polymères anioniques différents des carboxyalkylamidons, les polymères non ioniques, les polymères amphotères, les épaississants polymères associatifs ou non, les épaississants non polymères, les synergistes de mousses tels que des 1,2-alcanediols en C₁₀-C₁₈ ou des alcanolamides gras dérivés de mono ou de diéthanolamine d'acide gras en C8-C24, les colorants ou les pigments, les parfums, les huiles minérales, végétales ou synthétiques, les cires, les céramides, les alcools gras, les alcool gras oxyalkylénés, les vitamines, les provitamines notamment le panthénol, les filtres UV, les agents anti-radicalaires, les antipelliculaires, les agents anti-sebborrhéïques, les agents antichute des cheveux les conservateurs, les agents de stabilisation de pH, le carbonate de calcium, le talc , les argiles, le nitrure de bore, les solvants, et leurs mélanges et tout autre additif classiquement utilisé dans le domaine cosmétique qui n'affecte pas les propriétés des compositions selon l'invention.

Les compositions conformes à l'invention peuvent contenir en plus de l'association définie ci-dessus des agents régulateurs de viscosité tels que des électrolytes. On peut citer en particulier le chlorure de sodium, le xylène sulfonate de sodium, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir de préférence jusqu'à 5 % d'agents nacrants ou opacifiants bien connus dans l'état de la technique tels que par exemple les alcools gras supérieurs à C16 (alcool cétylique, alcool stéarylique, alcool béhénylique), les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

L'homme du métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Ces compositions peuvent se présenter sous la forme de crèmes, de mousses, de gel ou de pâtes et elles conviennent principalement au lavage des cheveux.

Les compositions peuvent être conditionnées sous diverses formes notamment dans des flacons, des flacons pompe, des flacons doseurs, dans des produits de conditionnement et de distribution à pompe sans reprise d'air.

Selon un mode de préparation des compositions selon l'invention, on mélange les composés à l'exception de l'amidon, on fait gonfler de préférence partiellement l'amidon dans de l'eau, puis on introduit cet amidon dans le reste de la composition et l'on mélange jusqu'à homogénéisation complète.

La présente invention concerne également un procédé de lavage des matières kératiniques qui consiste à appliquer une quantité efficace d'une composition telle que décrite ci-dessus, sur les matières kératiniques, à effectuer un rinçage par exemple avec de l'eau après un éventuel temps de pose.

Les exemples suivants sont donnés à titre illustratif de la présente invention. Toutes les quantités indiquées sont exprimées en % en poids, sauf indication contraire.

### Exemples 1 et 2

On a préparé les compositions de shampooing suivantes :

| Composition | 1 | 2 |
|---|---|---|
| Lauryléthersulfate de sodium (C12/C14 à 70/30) à 2.2 moles d'oxyde d'éthylène | 15 g MA | 13,5 g MA |
| Coco bétaïne (Mirataine BB/FLA de RHODIA) | 2,4 g MA | 2,4 g MA |
| Carboxyméthylamidon de sodium réticulé, (Primojel de DMV INTERNATIONAL) | 10 g | 10 g |
| Polyquaternium-10 (UCARE POLYMER JR400 LT de AMERCHOL) | | 0,5 gMA |
| Conservateurs, parfum | qs | qs |
| Acide citrique q.s. | pH 7 | pH 7 |
| Eau déminéralisée q.s.p. | 100 g | 100 g |

| | | |
|---|---|---|
| *: en Matière Active (MA) | | |

Les compositions conformes à l'invention ont un aspect de gel épais homogène et lisse.
Appliquée sur les cheveux humides, ces compositions génèrent une mousse abondante et onctueuse.
Les cheveux traités avec les compositions des exemples 1 et 2 sont doux et lisses et se démêlent facilement.

### EXEMPLES 3 à 5

On a préparé les compositions de shampooing suivantes :

| Composition | 3 | 4 | 5 |
|---|---|---|---|
| Lauryléthersulfate de sodium (C12/C14 à 70/30 en poids) à 2.2 moles d'oxyde d'éthylène | 13,5g MA | 13,5g MA | 13,5 g MA |
| Coco bétaïne (Mirataine BB/FLA de RHODIA) | 2,4g MA | 2,4g MA | 2,4g MA |
| Carboxyméthylamidon de sodium réticulé, (Primojel de DMV INTERNATIONAL) | 8 g | 6 g | 10 g |
| Chlorure d'hydroxypropyl guar triméthylammonium (Jaguar C13S de RHODIA) | 0,2 g MA | 0,15 g MA | 0,5 g MA |
| Polydiméthylsiloxane en émulsion (DC2-1691 de DOW CORNING) | | | 2,5 gMA |
| Conservateurs, parfum | qs | qs | qs |
| Acide citrique q.s. | pH 7 | pH 7 | pH 7 |
| Eau déminéralisée q.s.p. | 100 g | 100 g | 100 g |

Les compositions conformes à l'invention ont un aspect de gel épais homogène et lisse.
Appliquée sur les cheveux humides, ces compositions génèrent une mousse abondante et onctueuse.
Les cheveux traités avec les compositions des exemples 3 à 5 sont doux et lisses et se démêlent facilement.

### EXEMPLES 6 -7

On a préparé les compositions de shampooing suivantes :

| | 6 | 7 |
|---|---|---|
| Decyl polyglucoside (1,4) en solution aqueuse à 40% MA (Mydol 10 - KAO) | 10 g MA | 10 g MA |
| Polyquaternium 10 (Ucare polymer JR400 LT - AMERCHOL) | 0,5 g MA | --- |
| Homopolymère de chlorure de diallyl diméthyl ammonium (Merquat 100 de NALCO) | --- | 1,25 gMA |
| Carboxymethyl amidon de sodium (Primojel - DMV International) | 10 g | 10 g |
| Conservateurs | qs | qs |
| Parfum | qs | qs |
| Agent de pH | 7 ± 0,5 | 7 ± 0,5 |
| Eau déminéralisée qsp | 100 g | 100 g |

Les compositions conformes à l'invention présentent les m^mes propriétés que celles des exemples 1 à 5.

### EXEMPLES 8 à 10

On a préparé les compositions de shampooing suivantes :

| | 8 | 9 | 10 |
|---|---|---|---|
| Decyl polyglucoside (1,4) (Mydol 10 - KAO) | 10 g MA | 10 g MA | 10 g MA |
| Polyquaternium 10 (Ucare polymer JR400 LT - AMERCHOL) | 0,5 g MA | --- | --- |
| Homopolymère de chlorure de diallyl diméthyl ammonium (Merquat 100 de NALCO) | --- | 1,25 gMA | 1,25 gMA |
| Carboxymethyl amidon de sodium (Primojel - DMV International) | 10 g | 12 g | 12 g |
| Polydiméthylsiloxane (DC 2-1691 emulsion - DOW CORNING) | 3g MA | 3,5 g MA | --- |
| Amodimethicone en émulsion (DC 939 emulsion - Dow Corning) | --- | --- | 3,5 g MA |
| Conservateurs | qs | qs | qs |
| Parfum | qs | qs | qs |
| Agent de pH | 7 ± 0,5 | 7 ± 0,5 | 7 ± 0,5 |
| Eau déminéralisée qsp | 100 g | 100 g | 100 g |

Les compositions conformes à l'invention présentent les mêmes propriétés que celles des exemples 1 à 5.

## Revendications

1. Composition de lavage des matières kératiniques en particulier des fibres kératiniques, comprenant dans un milieu aqueux cosmétiquement acceptable :
- de 3 à 50 % en poids par rapport au poids total de la composition d'au moins un ou plusieurs tensioactifs choisis parmi les tensioactifs anioniques, les tensioactifs non ioniques et leurs mélanges,
- de plus de 5 % à 20 % en poids par rapport au poids total de la composition d'au moins un carboxyalkylamidon ou l'un de ses sels,
- de 50 à 92 % en poids d'eau par rapport au poids total de la composition,
le pH de la composition allant de 3 à 8.

2. Composition selon la revendication 1, **caractérisée en ce que** le carboxyalkylamidon est un carboxyalkyl(C1-C4) amidon.

3. Composition selon l'une quelconque des revendications précédentes, **caractérisée en ce que** les carboxyalkylamidons comprennent des motifs de formule suivante: 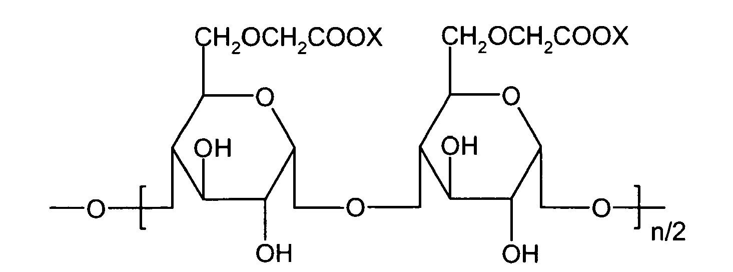 X désigne un atome d'hydrogène, un métal alcalin ou alcalinoterreux, NH₄, un ammonium quaternaire ou une amine organique.

4. Composition selon l'une quelconque des revendications précédentes, **caractérisée en ce que** les carboxyalkylamidons sont choisis parmi les carboxyméthylamidons de pomme de terre.

5. Composition selon l'une quelconque des revendications précédentes, **caractérisée en ce que** les carboxyalkylamidons sont non-prégélatinisés.

6. Composition selon l'une quelconque des revendications précédentes, **caractérisée en ce que** les carboxyalkylamidons sont réticulés.

7. Composition selon l'une quelconque des revendications précédentes, **caractérisée en ce que** le carboxyalkylamidon est présent en une quantité allant de plus de 5 % à 15 % en poids par rapport au poids total de la composition, de préférence de 6 à 12 % en poids.

8. Composition selon l'une quelconque des revendications précédentes, **caractérisée en ce que** le tensioactif anionique est choisi parmi les alkylsulfates, les alkyléthersulfates polyoxyalkylénés comportant de 2 à 5 motifs oxyde d'éthylène, et les alkyléthercarboxylates, et leurs mélanges.

9. Composition selon l'une des revendications précédentes, **caractérisée en ce que** le ou les tensioactifs anioniques sont présents en une quantité comprise allant de 3 à 50% en poids, de préférence de 4 à 25 % en poids par rapport au poids total de la composition.

10. Composition selon l'une quelconque des revendications 1 à 9, **caractérisée en ce que** le tensioactif non ionique est choisi parmi les (alkyl en C₆₋₂₄)-polyglycosides.

11. Composition selon l'une quelconque des revendications 1 à 10, **caractérisée en ce que** le ou les tensioactifs non ioniques sont présents en une quantité totale allant de 0,01 à 20 % en poids, de préférence allant de 0,5 à 10 % en poids par rapport au poids total de la composition.

12. Composition selon l'une quelconque des revendications précédentes, **caractérisée en ce qu'**elle comprend en outre au moins un tensioactif amphotère.

13. Composition selon la revendication 12, **caractérisée en ce que** le tensioactif amphotère est choisi parmi les (alkyl en C₈₋₂₀)-bétaïnes, les (alkyl en C₈₋₂₀)amido(alkyle en C₂₋₈)-bétaïnes, les alkylamphodiacétates et leurs mélanges.

14. Composition selon l'une quelconque des revendications 12 et 13, **caractérisée en ce que** le ou les tensioactifs amphotères sont présents en une quantité totale allant de 0,01 à 20 % en poids, de préférence allant de 0,5 à 10 % en poids par rapport au poids total de la composition.

15. Composition selon l'une quelconque des revendications précédentes, **caractérisée en ce que** le milieu aqueux cosmétiquement acceptable est constitué d'eau, ou d'un mélange d'eau et d'au moins un solvant organique.

16. Composition selon la revendication 15 **caractérisée en ce que** le solvant organique est choisi parmi les alcools inférieurs en C₁-C₄ et les polyols.

17. Composition selon l'une quelconque des revendications précédentes, **caractérisée en ce que** la composition comprend en outre au moins un polymère cationique.

18. Composition selon la revendication 17, **caractérisée en ce que** les polymères cationiques sont choisis parmi les dérivés d'éther de cellulose quaternaires, les gommes de guar cationiques, les cyclopolymères cationiques, les polymères quaternaires de vinylpyrrolidone et de vinylimidazole, et leurs mélanges.

19. Composition selon la revendication 17 ou 18, **caractérisée en ce que** les polymères cationiques sont choisis parmi les homopolymères ou copolymères de sels de diméthyldiallylammonium.

20. Composition selon l'une des revendications 17 à 19, **caractérisée en ce que** le ou les polymères cationiques sont présents à des concentrations allant de 0,01 à 20 %, de préférence de 0,05 à 10 % et plus particulièrement de 0,1 à 5 % en poids par rapport au poids total de la composition.

21. Composition selon l'une quelconque des revendications précédentes, **caractérisée en ce que** la composition comprend en outre au moins une silicone.

22. Composition selon la revendication 21, **caractérisée en ce que** la ou les silicones sont présentes à des concentrations allant de 0,01 à 20 % en poids, de préférence de 0,05 à 10 % en poids, plus particulièrement de 0,1 à 5 % et encore plus préférentiellement de 0,5 à 3 % en poids par rapport au poids total de la composition.

23. Composition selon l'une quelconque des revendications précédentes, **caractérisée en ce qu'**elle comprend d'autres ingrédients choisis parmi les polymères anioniques différents des carboxyalkylamidons, les polymères non ioniques, les polymères amphotères, les épaississants polymères associatifs ou non, les épaississants non polymères, les synergistes de mousses tels que des 1,2-alcanediols en C₁₀-C₁₈ ou des alcanolamides gras dérivés de mono ou de diéthanolamine d'acide gras en C8-C24, les colorants ou les pigments, les parfums, les huiles minérales, végétales ou synthétiques, les cires, les céramides, les alcools gras, les alcool gras oxyalkylénés, les vitamines, les provitamines notamment le panthénol, les filtres UV, les agents anti-radicalaires, les antipelliculaires, les agents anti-sebborrhéïques, les agents antichute des cheveux, les conservateurs, les agents de stabilisation de pH, le carbonate de calcium, le talc , les argiles, le nitrure de bore, les solvants, les agents nacrants, les opacifiants, et leurs mélanges.

24. Utilisation cosmétique de la composition selon l'une quelconque des revendications précédentes, pour le lavage et le conditionnement des matières kératiniques et en particulier des fibres kératiniques.

25. Procédé cosmétique de lavage et de conditionnement des matières kératiniques et en particulier des fibres kératiniques, comprenant l'application d'une quantité efficace d'une composition selon l'une quelconque des revendications 1 à 23, sur les cheveux, et un rinçage après un éventuel temps de pose.

## Claims

1. Composition for washing keratin materials, in particular keratin fibres, comprising, in a cosmetically acceptable aqueous medium:
- from 3% to 50% by weight, relative to the total weight of the composition, of at least one or more surfactants chosen from anionic surfactants, nonionic surfactants and mixtures thereof,
- from more than 5% to 20% by weight, relative to the total weight of the composition, of at least one carboxyalkyl starch or a salt thereof,
- from 50% to 92% by weight of water, relative to the total weight of the composition,
the pH of the composition ranging from 3 to 8.

2. Composition according to Claim 1, **characterized in that** the carboxyalkyl starch is a carboxy(C1-C4)alkyl starch.

3. Composition according to either of the preceding claims, **characterized in that** the carboxyalkyl starches comprise units having the following formula: 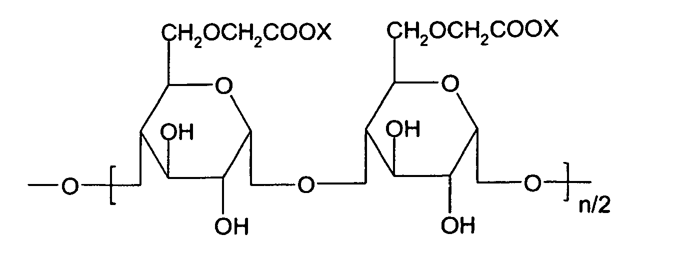 X denoting a hydrogen atom, an alkali metal or alkaline-earth metal, NH₄, a quaternary ammonium or an organic amine.

4. Composition according to any one of the preceding claims, **characterized in that** the carboxyalkyl starches are chosen from carboxymethyl potato starches.

5. Composition according to any one of the preceding claims, **characterized in that** the carboxyalkyl starches are non-pregelatinized.

6. Composition according to any one of the preceding claims, **characterized in that** the carboxyalkyl starches are crosslinked.

7. Composition according to any one of the preceding claims, **characterized in that** the carboxyalkyl starch is present in an amount ranging from more than 5% to 15% by weight and preferably from 6% to 12% by weight relative to the total weight of the composition.

8. Composition according to any one of the preceding claims, **characterized in that** the anionic surfactant is chosen from alkyl sulfates, polyoxyalkylenated alkyl ether sulfates comprising from 2 to 5 ethylene oxide units, and alkyl ether carboxylates, and mixtures thereof.

9. Composition according to one of the preceding claims, **characterized in that** the anionic surfactant(s) is (are) present in an amount ranging from 3% to 50% by weight and preferably from 4% to 25% by weight relative to the total weight of the composition.

10. Composition according to any one of Claims 1 to 9, **characterized in that** the nonionic surfactant is chosen from (C₆₋₂₄ alkyl)polyglycosides.

11. Composition according to any one of Claims 1 to 10, **characterized in that** the nonionic surfactant(s) is (are) present in a total amount ranging from 0.01% to 20% by weight and preferably ranging from 0.5% to 10% by weight relative to the total weight of the composition.

12. Composition according to any one of the preceding claims, **characterized in that** it also comprises at least one amphoteric surfactant.

13. Composition according to Claim 12, **characterized in that** the amphoteric surfactant is chosen from (C₈₋₂₀ alkyl)betaines, (C₈₋₂₀ alkyl)amido(C₂₋₈ alkyl)betaines and alkylamphodiacetates, and mixtures thereof.

14. Composition according to either of Claims 12 and 13, **characterized in that** the amphoteric surfactant(s) is (are) present in a total amount ranging from 0.01% tc 20% by weight and preferably ranging from 0.5% to 10% by weight relative to the total weight of the composition.

15. Composition according to any one of the preceding claims, **characterized in that** the cosmetically acceptable aqueous medium is constituted of water or of a mixture of water and of at least one organic solvent.

16. Composition according to Claim 15, **characterized in that** the organic solvent is chosen from C₁-C₄ lower alcohols and polyols.

17. Composition according to any one of the preceding claims, **characterized in that** the composition also comprises at least one cationic polymer.

18. Composition according to Claim 17, **characterized in that** the cationic polymers are chosen from quaternary cellulose ether derivatives, cationic guar gums, cationic cyclopolymers and quaternary vinylpyrrolidone and vinylimidazole polymers, and mixtures thereof.

19. Composition according to Claim 17 or 18, **characterized in that** the cationic polymers are chosen from homopolymers or copolymers of dimethyldiallylammonium salts.

20. Composition according to one of Claims 17 to 19, **characterized in that** the cationic polymer(s) is (are) present in concentrations ranging from 0.01% to 20%, preferably from 0.05% to 10% and more particularly from 0.1% to 5% by weight relative to the total weight of the composition.

21. Composition according to any one of the preceding claims, **characterized in that** the composition also comprises at least one silicone.

22. Composition according to Claim 21, **characterized in that** the silicone(s) is (are) present in concentrations ranging from 0.01% to 20% by weight, preferably from 0.05% to 10% by weight, more particularly from 0.1% to 5% and even more preferentially from 0.5% to 3% by weight relative to the total weight of the composition.

23. Composition according to any one of the preceding claims, **characterized in that** it comprises other ingredients chosen from anionic polymers other than carboxyalkyl starches, nonionic polymers, amphoteric polymers, associative or non-associative polymeric thickeners, non-polymeric thickeners, foam synergists such as C₁₀-C₁₈ 1,2-alkanediols or fatty alkanolamides derived from C₈-C₂₄ fatty acid monoethanolamine or diethanolamine, dyes or pigments, fragrances, mineral, plant or synthetic oils, waxes, ceramides, fatty alcohols, oxyalkylenated fatty alcohols, vitamins, provitamins in particular panthenol, UV-screening agents, free-radical scavengers, antidandruff agents, anti-seborrhoea agents, agents for preventing hair loss, preserving agents, pH stabilizers, calcium carbonate, talc, clays, boron nitride, solvents nacreous agents, opacifiers, and mixtures thereof.

24. Cosmetic use of the composition according to any one of the preceding claims, for washing and conditioning keratin materials, and in particular keratin fibres.

25. Cosmetic process for washing and conditioning keratin materials, and in particular keratin fibres, comprising the application of an effective amount of a composition according to any one of Claims 1 to 23 to the hair, and rinsing after an optional action time.

## Patentansprüche

1. Zusammensetzung für die Reinigung von Keratinsubstanzen und insbesondere Keratinfasern, die in einem kosmetisch akzeptablen wässrigen Medium enthält:
- 3 bis 50 Gew.-% mindestens eines oder mehrerer grenzflächenaktiver Stoffe, die unter den anionischen grenzflächenaktiven Stoffen, nichtionischen grenzflächenaktiven Stoffen und deren Gemische ausgewählt sind, bezogen auf das Gesamtgewicht der Zusammensetzung,
- mehr als 5 bis 20 Gew.-% mindestens einer Carboxyalkylstärke oder eines ihrer Salze, bezogen auf das Gesamtgewicht der Zusammensetzung,
- 50 bis 92 Gew.-% Wasser, bezogen auf das Gesamtgewicht der Zusammensetzung,
wobei der pH-Wert der Zusammensetzung im Bereich von 3 bis 8 liegt.

2. Zusammensetzung nach Anspruch 1, **dadurch gekennzeichnet, dass** die Carboxyalkylstärke eine Carboxyalkyl(C₁₋₄)stärke ist.

3. Zusammensetzung nach einem der vorhergehenden Ansprüche, **dadurch gekennzeichnet, dass** die Carboxyalkylstärken Einheiten der folgenden Formel enthalten: 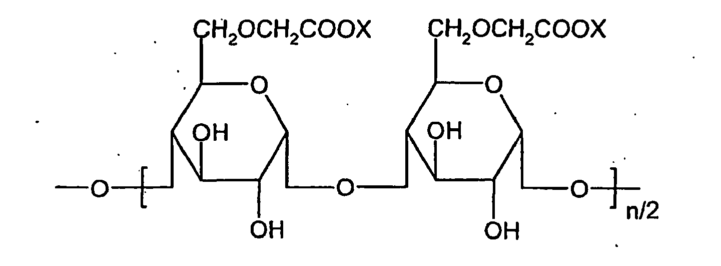 X bedeutet ein Wasserstoffatom, ein Alkalimetall oder ein Erdalkalimetall, NH₄, eine quartäre Ammoniumgruppe oder ein organisches Amin.

4. Zusammensetzung nach einem der vorhergehenden Ansprüche, **dadurch gekennzeichnet, dass** die Carboxyalkylstärken unter den Carboxymethylstärken aus der Kartoffel ausgewählt sind.

5. Zusammensetzung nach einem der vorhergehenden Ansprüche, **dadurch gekennzeichnet, dass** die Carboxyalkylstärken nicht vorverkleistert sind.

6. Zusammensetzung nach einem der vorhergehenden Ansprüche, **dadurch gekennzeichnet, dass** die Carboxyalkylstärken vernetzt sind.

7. Zusammensetzung nach einem der vorhergehenden Ansprüche, **dadurch gekennzeichnet, dass** die Carboxyalkylstärke in einem Mengenanteil von mehr als 5 bis 15 Gew.-%, bezogen auf das Gesamtgewicht der Zusammensetzung, und vorzugsweise 6 bis 12 Gew.-% enthalten ist.

8. Zusammensetzung nach einem der vorhergehenden Ansprüche, **dadurch gekennzeichnet, dass** der anionische grenzflächenaktive Stoff unter den Alkylsulfaten, Alkylethersulfaten, die polyalkoxyliert sind und 2 bis 5 Einheiten Ethylenoxid enthalten, und Alkylethercarboxylaten und deren Gemischen ausgewählt ist.

9. Zusammensetzung nach einem der vorhergehenden Ansprüche, **dadurch gekennzeichnet, dass** der oder die anionischen grenzflächenaktiven Stoffe in einem Mengenanteil im Bereich von 3 bis 50 Gew.-% und vorzugsweise 4 bis 25 Gew.-%, bezogen auf das Gesamtgewicht der Zusammensetzung, enthalten sind.

10. Zusammensetzung nach einem der Ansprüche 1 bis 9, **dadurch gekennzeichnet, dass** der nichtionische grenzflächenaktive Stoff unter den Alkyl(C₆₋₂₄)polyglycosiden ausgewählt ist.

11. Zusammensetzung nach einem der Ansprüche 1 bis 10, **dadurch gekennzeichnet, dass** der oder die nichtionischen grenzflächenaktiven Stoffe in einer Gesamtmenge von 0,01 bis 20 Gew.-% und vorzugsweise 0,5 bis 10 Gew.-%, bezogen auf das Gesamtgewicht der Zusammensetzung, vorliegen.

12. Zusammensetzung nach einem der vorhergehenden Ansprüche, **dadurch gekennzeichnet, dass** sie ferner mindestens einen amphoteren grenzflächenaktiven Stoff enthält.

13. Zusammensetzung nach Anspruch 12, **dadurch gekennzeichnet, dass** der amphotere grenzflächenaktive Stoff unter den Alkyl(C₈₋₂₀)betainen, Alkyl(C₈₋₂₀)amidoalkyl(C₂₋₈)betainen, Alkylamphodiacetaten und deren Gemische ausgewählt ist.

14. Zusammensetzung nach einem der Ansprüche 12 und 13, **dadurch gekennzeichnet, dass** der oder die amphoteren grenzflächenaktiven Stoffe in einer Gesamtmenge von 0,01 bis 20 Gew.-% und vorzugsweise 0,5 bis 10 Gew.-%, bezogen auf das Gesamtgewicht der Zusammensetzung, vorliegen.

15. Zusammensetzung nach einem der vorhergehenden Ansprüche, **dadurch gekennzeichnet, dass** das kosmetisch akzeptable wässrige Medium aus Wasser oder einem Gemisch aus Wasser und mindestens einem organischen Lösungsmittel besteht.

16. Zusammensetzung nach Anspruch 15, **dadurch gekennzeichnet, dass** das organische Lösungsmittel unter den niederen Alkoholen mit 1 bis 4 Kohlenstoffatomen und Polyolen ausgewählt ist.

17. Zusammensetzung nach einem der vorhergehenden Ansprüche, **dadurch gekennzeichnet, dass** die Zusammensetzung ferner mindestens ein kationisches Polymer enthält.

18. Zusammensetzung nach Anspruch 17, **dadurch gekennzeichnet, dass** die kationischen Polymere unter den quartären Celluloseetherderivaten, kationischen Guargummen, kationischen Cyclopolymeren, quartären Polymeren von Vinylpyrrolidon und Vinylimidazol und deren Gemischen ausgewählt sind.

19. Zusammensetzung nach Anspruch 17 oder 18, **dadurch gekennzeichnet, dass** die kationischen Polymere unter den Homopolymeren oder Copolymeren von Dimethyldiallylammoniumsalzen ausgewählt sind.

20. Zusammensetzung nach einem der Ansprüche 17 bis 19, **dadurch gekennzeichnet, dass** das oder die kationischen Polymere in Konzentrationen von 0,01 bis 20 %, vorzugsweise 0,05 bis 10 % und insbesondere 0,1 bis 5 Gew.-%, bezogen auf das Gesamtgewicht der Zusammensetzung, vorliegen.

21. Zusammensetzung nach einem der vorhergehenden Ansprüche, **dadurch gekennzeichnet, dass** die Zusammensetzung ferner mindestens ein Silicon enthält.

22. Zusammensetzung nach Anspruch 21, **dadurch gekennzeichnet, dass** das oder die Silicone in Konzentrationen von 0,01 bis 20 Gew.-%, vorzugsweise 0,05 bis 10 Gew.-%, besonders 0,1 bis 5 % und noch bevorzugter 0,5 bis 3 Gew.-%, bezogen auf das Gesamtgewicht der Zusammensetzung, vorliegen.

23. Zusammensetzung nach einem der vorhergehenden Ansprüche, **dadurch gekennzeichnet, dass** sie ferner weitere Bestandteile enthält, die unter den anionischen Polymeren, die von Carboxyalkylstärken verschieden sind, nichtionischen Polymeren, amphoteren Polymeren, assoziativen oder nichtassoziativen Polymeren Verdickungsmitteln, nichtpolymeren Verdickungsmitteln, für die Schaumbildung synergistischen Stoffen, wie 1,2-Alkandiolen mit 10 bis 18 Kohlenstoffatomen oder Alkanolfettamiden, die von Fettsäure(C₈₋₂₄)mono- oder -diethanolamin abgeleitet sind, Farbstoffen oder Pigmenten, Parfums, Mineralölen, pflanzlichen Ölen oder synthetischen Ölen, Wachsen, Ceramiden, Fettalkoholen, alkoxylierten Fettalkoholen, Vitaminen, Provitaminen, besonders Panthenol, UV-Filtern, Radikalfängern für freie Radikale, Antischuppenmitteln, Wirkstoffen gegen Seborrhoe, Wirkstoffen gegen Haarausfall, Konservierungsmitteln, pH-Stabilisatoren, Calciumcarbonat, Talk, Tonen, Bornitrid, Lösungsmitteln, Perlglanzstoffen, Trübungsmitteln und deren Gemischen ausgewählt sind.

24. Kosmetische Verwendung der Zusammensetzung nach einem der vorhergehenden Ansprüche für die Reinigung und Konditionierung von Keratinsubstanzen und insbesondere Keratinfasern.

25. Kosmetisches Verfahren für die Reinigung und Konditionierung von Keratinsubstanzen und insbesondere Keratinfasern, das das Aufbringen einer wirksamen Menge einer Zusammensetzung nach einem der Ansprüche 1 bis 23 auf die Haare und, nach einer gegebenenfalls abgewarteten Einwirkzeit, einen Spülschritt umfasst.
